# EUROPEAN PATENT APPLICATION

(11) **EP 4 645 333 A1**
(43) Date of publication of application: **05.11.2025**
(21) Application number: 24305688.4
(22) Date of filing: 30.04.2024
(51) Int. Cl.: G16H 30/40, A61B 34/10, G06F 3/0346, G06F 3/04812, G06F 3/04815, G06T 15/08, G06T 19/20, G16H 50/00

(54) **DEVICE AND METHOD FOR ASSISTING A USER POSITIONING AT LEAST ONE GRAPHICAL LANDMARK WITHIN A 3D MODEL**

(71) Applicant: Avatar Medical, 75015 Paris (FR)
(72) Inventor: EL BEHEIRY, Mohamed, 75015 PARIS (FR); BRIENT-LITZLER, Elodie, 75015 PARIS (FR)
(74) Representative: Icosa

(57) **Abstract**

The present invention relates to a device and a method for assisting a user (4) positioning at least one graphical landmark within a three-dimensional - 3D - model by means of a user graphical interface (2) and a controller (6) configured so that the user (4) interacts with said user graphical interface (2), said 3D model being obtained from at least one 3D image and being representative of at least one portion of a patient's body and comprising at least one anatomical element of interest on which the at least one graphical landmark is to be positioned.

## Description

### FIELD OF INVENTION

The present invention relates to the field of medical imaging, particularly to a method and a device for assisting a user positioning at least one graphical within a three-dimensional - 3D - model.

### BACKGROUND OF INVENTION

In the realm of modern surgical practice, the ability to accurately identify and delineate anatomical landmarks plays a pivotal role in ensuring the success and safety of medical interventions. As surgeons navigate the complexities of patient anatomy to plan and execute procedures with precision, the need for reliable methodologies to position landmarks on three-dimensional (3D) patient models has become increasingly apparent. This patent application endeavors to address this critical challenge by presenting a novel approach for the accurate placement of landmarks on 3D patient models during preoperative planning stages, thereby enhancing surgical precision and optimizing patient outcomes.

Traditional methods of landmark identification and localization in surgical planning have often relied on manual techniques or simplistic digital tools that fail to capture the intricacies of patient-specific anatomy. While medical imaging modalities such as computed tomography (CT), magnetic resonance imaging (MRI), and three-dimensional reconstructions have revolutionized our ability to visualize internal structures, the translation of this information into actionable insights for surgical planning remains fraught with challenges.

The central question driving this patent application is as follows: How can we develop a methodology and system that enable surgeons to accurately position landmarks on 3D patient models, accounting for variations in patient anatomy, surgical approach, and procedural objectives?

By posing this question, we recognize the multifaceted nature of the challenge at hand. From orthopedic procedures necessitating precise identification of joint surfaces and bony prominences to neurosurgical interventions requiring localization of critical neural structures, the accurate placement of landmarks serves as the cornerstone of effective surgical planning and intraoperative navigation.

Our proposed solution seeks to harness the power of advanced imaging technologies, computational algorithms, and user-friendly interfaces to streamline the process of landmark positioning on 3D patient models. By integrating automated rendering techniques, relief and depth visualization techniques, as well as segmentation techniques and or machine learning algorithms, our system aims to empower surgeons with intuitive tools for identifying and annotating landmarks with submillimeter accuracy, facilitating comprehensive preoperative planning and intraoperative guidance.

In essence, this patent application represents a pioneering effort to bridge the gap between medical imaging and surgical practice by introducing a robust framework for landmark positioning on 3D patient models. Through a combination of innovation, precision, and usability, our methodology aims to revolutionize the way surgeons plan and execute procedures, ultimately improving patient outcomes and advancing the field of surgical care.

### SUMMARY

To this end, the present disclosure relates to a computer-implemented method for assisting a user positioning at least one graphical landmark within a three-dimensional - 3D - model by means of a user graphical interface and a controller configured so that the user interacts with the user graphical interface. The 3D model has been previously obtained from at least one 3D image and is representative of at least one portion of a patient's body. Furthermore, the 3D model comprises at least one anatomical element of interest on which the at least one graphical landmark is to be positioned,

The method comprises:
- computing a surface of the at least one anatomical element of interest;
- computing a first 3D rendering of the surface by using a 3D rendering technique;
- displaying, in the user graphical interface, a virtual scene comprising the first 3D rendering of the surface on which is overlaid a virtual representation of the controller;
- in the virtual scene, displaying on the surface a spot indicator providing a visual representation of distances between one reference point on the surface, the reference point being selected by the user using the controller, and surrounding pixels belonging to the surface, wherein displaying the spot indicator provides a local displaying enhancement of reliefs on the at least one anatomical element of interest represented by the surface;
wherein the controller is further configured to interactively move the spot indicator on the surface, as guidance for the user to identify anatomical landmarks of interest, and to position the at least one graphical landmark to a desired localization on the surface.

Such a method enables precise identification and localization of landmarks on the 3D model, ensuring that surgical plans are based on accurate anatomical data. In fact, positioning graphical landmarks on a 3D model during surgical planning offers numerous benefits, including improved anatomical localization, enhanced spatial orientation, optimized surgical route planning, intraoperative guidance, customized surgical approaches, and enhanced communication and collaboration. By leveraging 3D models with annotated landmarks, surgeons can optimize preoperative planning, streamline intraoperative workflows, and ultimately improve patient outcomes.

Furthermore, it is to be noted that this method works well if the virtual scene is displayed on a 3D display device (such as stereoscopic screens or virtual and augmented reality head mounted displays for example), but also on a 2D display device where it is much more difficult to have a precise visual rendering of the relief or depth of a real anatomical element (due to "flat" 3D rendering) than on a 3D display device. This precision reduces the margin of error during surgery, leading to improved outcomes and reduced risks of complications.

By allowing surgeons to annotate patient-specific landmarks on the 3D model, the method facilitates personalized surgical planning tailored to the unique anatomy of each patient (e.g. for selecting the most suitable implant according to the shape of the bones and their density, specific features of the surrounding anatomical environment). This individualized approach increases the likelihood of successful outcomes and minimizes the need for intraoperative adjustments. Furthermore, accurately positioned landmarks serve as navigational guides during surgery, streamlining the surgical workflow and reducing the time required for intraoperative decision-making. This optimization of procedural efficiency not only benefits the patient by minimizing anesthesia and operative durations but also enhances resource utilization within the healthcare system. The method also fosters effective communication and collaboration among members of the surgical team by providing a standardized platform for annotating and sharing landmark positions on the 3D model. This enhanced communication reduces the likelihood of misunderstandings or discrepancies during surgical planning and execution.

Moreover, by enabling precise landmark positioning on the 3D model, the method opens doors to the implementation of novel surgical techniques that rely on accurate anatomical guidance. Surgeons can explore innovative approaches and interventions with confidence, knowing that they have a reliable tool for navigating complex anatomical structures.

Finally, the method also may serve as a valuable training and educational tool for surgical trainees and medical students, allowing them to practice landmark identification and surgical planning in a simulated environment. This hands-on experience enhances learning outcomes and prepares future surgeons for real-world clinical scenarios.

According to other advantageous aspects of the invention, the method comprises one or more of the features described in the following embodiments, taken alone or in any possible combination.

The 3D rendering technique may be a volume rendering technique, such as raycasting or raytracing, which enables to produce highly realistic and accurate renderings of the 3D model, capturing details such as shading, transparency, and occlusion. This fidelity enables medical professionals to analyze complex structures with precision. These techniques offer flexibility, enabling users to adjust parameters such as transfer functions, lighting, and viewing angles to customize the visualization to their specific needs. Moreover, raycasting and ray tracing techniques preserve spatial relationships within the volume. This preservation of depth cues and spatial context is crucial for accurate interpretation and analysis of complex 3D models. Raycasting and ray tracing can also seamlessly integrate multi-modal imaging data, such as combining CT and MRI scans or overlaying functional data onto anatomical structures, enabling comprehensive visualization and analysis of complex 3D models.

The reference point may be positioned on the surface at an intersection between a line, passing between a virtual position associated to the user's eyes in the virtual scene and the virtual representation of the controller, and the surface. Such positioning has the advantage to be easily applicable whether with a 2D display device (where the controller may be a mouse for example) or with a 3D display device (where the controller may be a 3D controller).

Computing the surface of the at least one anatomical element of interest may comprise executing a segmentation algorithm for segmenting the surface of the at least one anatomical element of interest. Segmenting the surface allows for the creation of a highly detailed and accurate first 3D rendering of the surface. This precision facilitates better visualization and understanding of complex anatomical elements, such as organs, bones, and soft tissues, as well as a more precise positioning of the at least one graphical landmark.

The spot indicator on the surface may be visualized by at least one displaying characteristic taken among: color, intensity, transparency, opacity. These characteristics allow the user to optimize and personalize the visualization of the relief and of the depth of the first 3D rendering of the surface displayed at the virtual representation of the controller.

In some embodiments, the virtual scene can be displayed on a 2D display device such as, for example: a computer monitor, a tablet, a smartphone, or a projection screen. The method may further comprise at least one of the following features:

The at least one displaying characteristics of the spot indicator may be calculated using a distance between the selected reference point on said surface and the surrounding pixels belonging to the surface within a predefined spot region approximately centered on the reference point. In one embodiment, the spot indicator could be a colored heat-map overlaid with the surface to represent these distances. These additional characteristics can help the user better assess the local topology of the surface than with the sole raycasting or raytracing effects, so as to position the landmark more precisely.

The surface is represented by a mesh composed of vertices, edges, and faces defining a shape of the at least one anatomical element of interest, and selecting the reference point on the surface using the controller comprises:
- calculating a position of the virtual representation of the controller in the virtual scene;
- identifying a vertex of the mesh as being the closest vertex to the calculated position of the controller virtual representation;
- selecting the identified vertex as the reference point on the surface.

Using 3D displays and 3D controllers, the manual positioning of a landmark is typically achieved by positioning the controller at the coordinates where the landmark should be positioned. Choosing a landmark would consist in moving the controller until the virtual representation of the controller is located on the reference point. The user would move the controller until they see the spot indicator being located and centered around the position where they want to position the landmark. As a consequence, they would have a direct enhanced visual feedback on the position of their controller leading to more precise positioning.

In some embodiments, the virtual scene can be displayed on a 3D display device such as, for example: a stereoscopic 3D monitor, an autostereoscopic 3D display, a virtual reality - VR - headset, an augmented reality - AR - device, or a 3D holographic display. The method may further comprise at least one of the following features:

Computing the surface comprises executing a ray casting or ray tracing algorithm on the at least one 3D image to obtain a 3D patient rendering, and a depth map and/or a position map associated to the 3D patient rendering, the surface being obtained based on the depth map and/or the position map, and wherein displaying on the surface the spot indicator depends on the depth map and/or the position map. This embodiment is particularly interesting to interact with a volume rendering on a 2D display with a mouse controller, in the context where the positioning of a landmark at the right depth might be challenging. Indeed, overlaying a mouse cursor representation on the 3D volume rendering does not provide an indication on the depth with which the landmark should be positioned. The depth map and/or the position map, which is generated along the execution of the ray casting or ray tracing, can be used as a surface in our current invention to determine this 3D positioning in space.

The at least one displaying characteristic of the spot indicator is obtained by including a local light source during computation of 3D rendering, by:
- determining a current position of the local light source from the reference point selected by means of the controller;
- computing a current 3D rendering of the surface considering the local light source;
wherein, as the reference point selected by means of the controller changes as a result of the interactions of the user with the controller, the current 3D rendering is recomputed taking into account the current position of the local light source. This light source creates local shadows and reflections enhancing the perception of the local topology of the surface.

The virtual representation of the controller is a 3D object, such as a 3D cross or a 3D landmark.

In some embodiments compatible with displaying the virtual scene on a 2D or a 3D display device, the method may further comprise at least one of the following technical features:

Recording the coordinates corresponding to the position of the at least one graphical landmark on the 3D model into a memory. The coordinates can then be used for computing measurements. They can be exported within an exported file. They can be retrieved by the user during a second planning session.

Computing measurements using the coordinates of at least two graphical landmarks. Measurements can be key distances or angles that reflect pathological or healthy conditions, and be used to analyze the case and plan the best treatment strategy.

Receiving initial predefined localization of at least one of the anatomical landmarks of interest associated to the 3D model, and displaying, in the virtual scene, on the surface at least one guiding landmark corresponding to the initial predefined localization, wherein the controller is configured to move the at least one guiding landmark onto another localization on the surface. This leads to the possibility of editing an initially placed landmark, which leaves room for initial error of judgment or imprecision with the controller.

According to a second aspect, the present disclosure relates to a use of recorded coordinates with the method according to the previous embodiments, for positioning a medical device, such as a surgical device, an imaging device or an implant as relative to these coordinates during a medical intervention.

According to a third aspect, the present disclosure relates to a device having a user graphical interface, a controller, configured to control the interaction between a user and the user graphical interface, and at least one processor configured to carry out a method for assisting a user positioning at least one graphical landmark within a three-dimensional - 3D - model by means of a user graphical interface according to any one of the embodiments previously described.

The preferential and advantageous characteristics linked to the method previously described are also applicable to the device.

Furthermore, the controller may be one of: a mouse, a trackpad, a joystick for virtual reality headset, depending on the type of display device used to display the virtual scene (i.e. 2D or 3D display device).

According to a fourth aspect, the present disclosure relates to a computer program comprising software code adapted to perform a method for assisting a user positioning at least one graphical landmark within a three-dimensional - 3D - model by means of a user graphical interface compliant with any of the above execution modes when the program is executed by a processor.

According to a fifth aspect, the present disclosure relates to a non-transitory program storage device, readable by a computer, tangibly embodying a program of instructions executable by the computer to perform a method for assisting a user positioning at least one graphical landmark within a three-dimensional - 3D - model by means of a user graphical interface compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:
The terms **"adapted"** and **"configured"** are used in the present disclosure as broadly encompassing initial configuration, later adaptation or complementation of the present device, or any combination thereof alike, whether effected through material or software means (including firmware).
The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.
**"Machine learning (ML)"** designates in a traditional way computer algorithms improving automatically through experience, on the ground of training data enabling to adjust parameters of computer models through gap reductions between expected outputs extracted from the training data and evaluated outputs computed by the computer models.

Additional terms will be defined, specified or commented wherever useful throughout the following description.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:

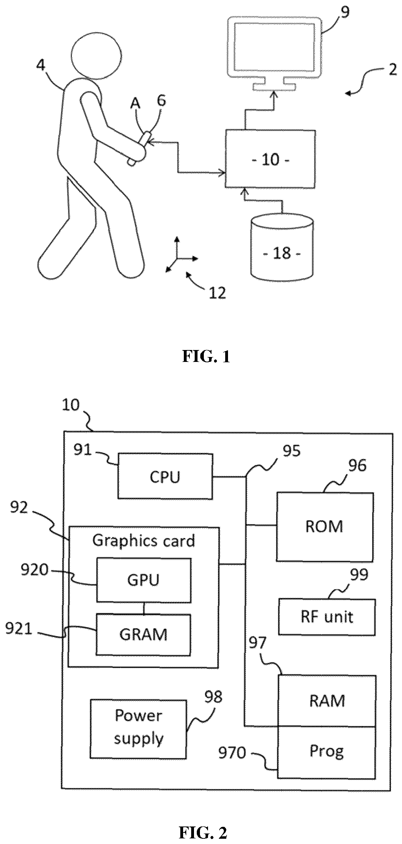

**Figure 1** is a schematic representation of a device configured to implement the method according to embodiments of the present invention.
**Figure 2** is a schematic representation of a processing unit of the device of Figure 1.

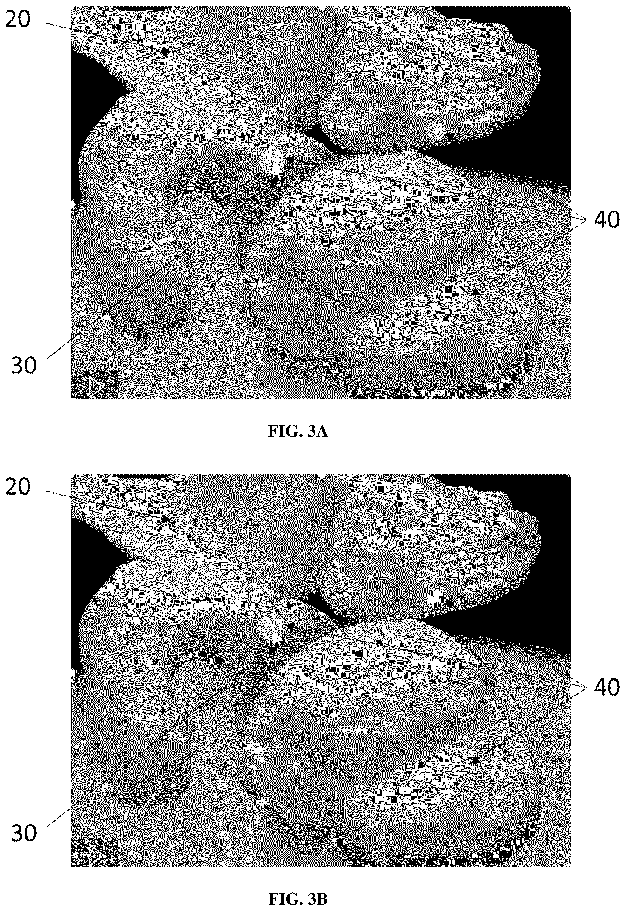

**Figure 3A** (which is in color) and **Figure 3B** (which is the black and white version of figure 3A) represent a view of a virtual scene comprising a 3D model on which graphical landmarks are being positioned according to an example embodiment of a method for assisting a user positioning at least one graphical landmark within a three-dimensional - 3D - model by means of a user graphical interface and a controller configured so that the user interacts with the user graphical interface.

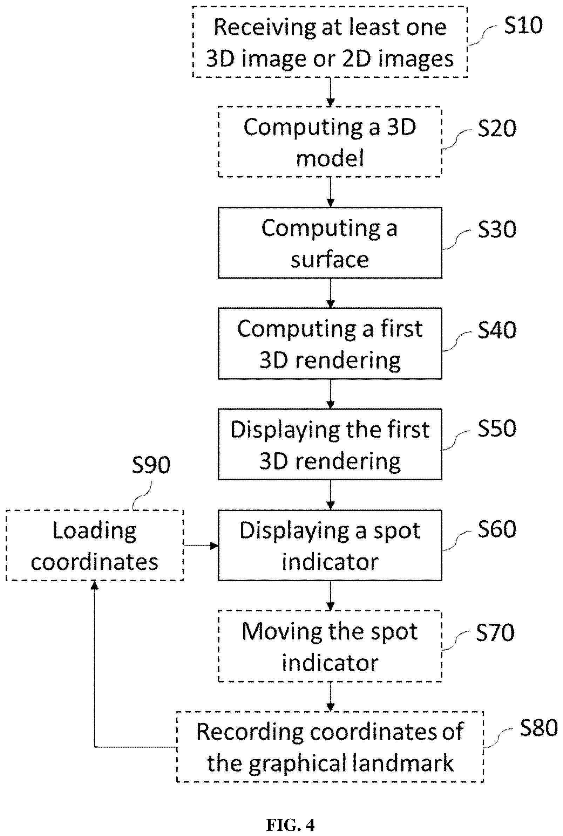

**Figure 4** is an example of flow chart of the method for assisting a user positioning at least one graphical landmark within a three-dimensional - 3D - model by means of a user graphical interface and a controller configured so that the user interacts with the user graphical interface.

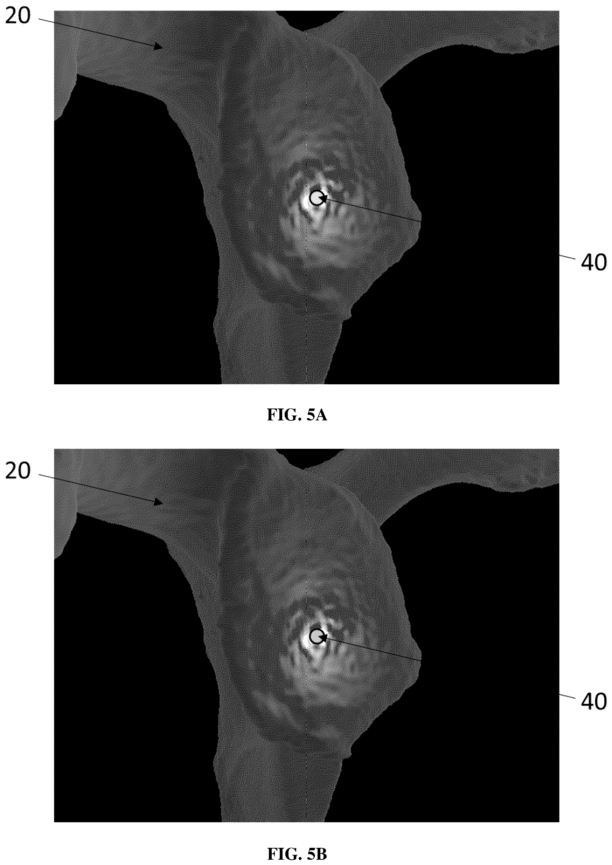

**Figure 5A** (which is in color) and **Figure 5B** (which is the black and white version of figure 5A) represent a view of a virtual scene comprising a 3D model on which a graphical landmark represented by a point light source has been positioned according to an example embodiment of the present method.

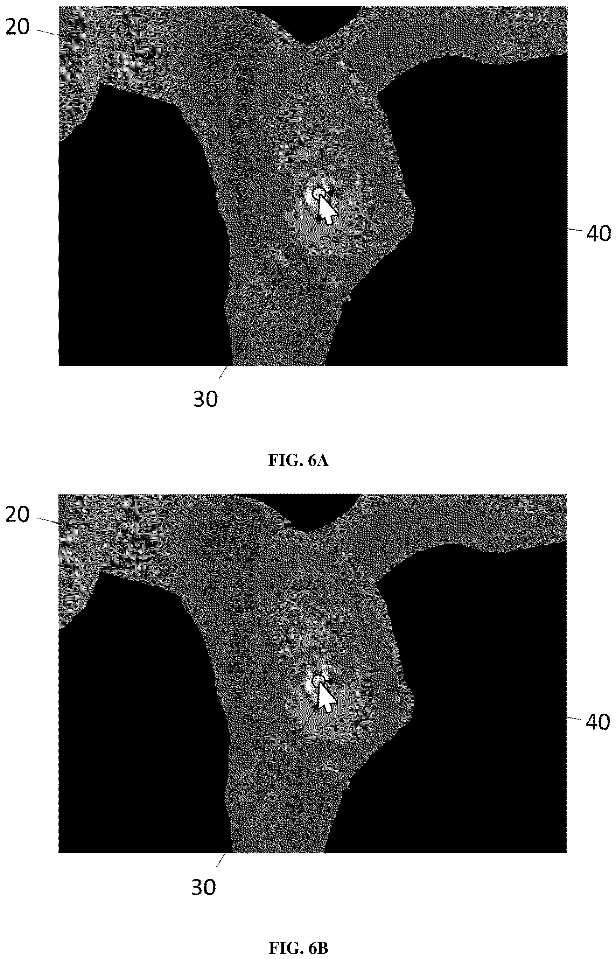

**Figure 6A** (which is in color) and **Figure 6B** (which is the black and white version of figure 6A) represent a view of the point light source of figures 4A and 4B which can be selected and moved with a controller represented by a mouse cursor according to an example embodiment of the present method.

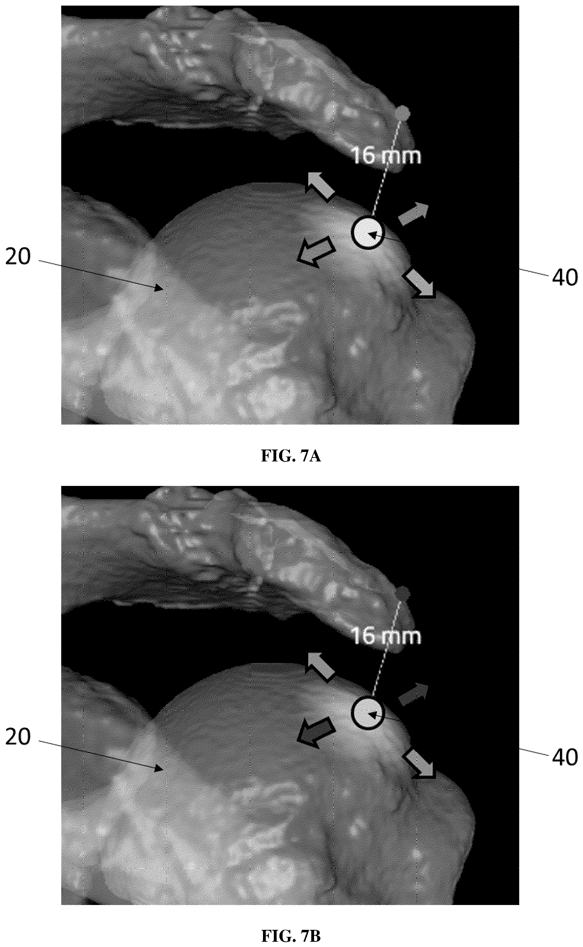

**Figure 7A** (which is in color) and **Figure 7B** (which is the black and white version of figure 7A) represent a view of a virtual scene comprising a 3D model on which graphical landmarks represented by point light sources can be positioned and moved by a gizmo according to an example embodiment of the present method, and with which anatomical measurements can be made.

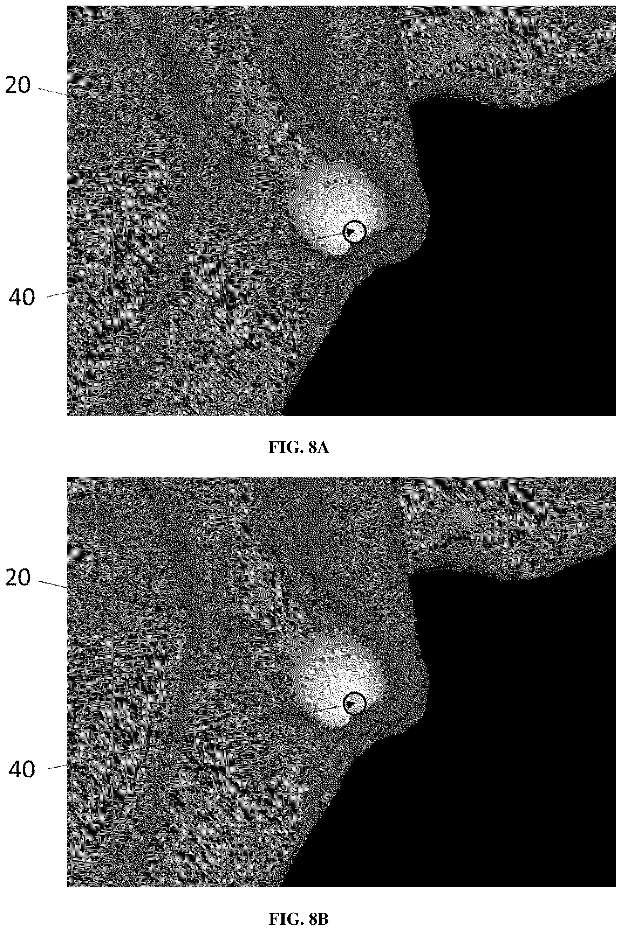

**Figure 8A** (which is in color) and **Figure 8B** (which is the black and white version of figure 8A) represent a view of a virtual scene comprising a 3D model on which a graphical landmark represented by a point light source is positioned and locally modifies the color of the surface on which it is positioned depending on the surface's depth and relief, according to an example embodiment of the present method.

### DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

The invention relates to a computer-implemented method for assisting a user positioning at least one graphical landmark within a three-dimensional - 3D - model by means of a user graphical interface and a controller configured so that the user interacts with the user graphical interface, the 3D model being obtained from at least one 3D image and being representative of at least one portion of a patient's body and comprising at least one anatomical element of interest on which the at least one graphical landmark is to be positioned.

The at least one anatomical element of interest may be one of: a specific bone or an assembly of bones forming a particular structure (such as, for example: a shoulder, a knee, a hip, an elbow, or wrist joints), soft tissues (such as, for example: muscles, tendons, ligaments, cartilage) or organs.

This method may be implemented by a device as illustrated in Figure 1.

**Figure 1** is a schematic representation of a device configured to implement embodiments of a method for assisting a user 4 positioning at least one graphical landmark within a three-dimensional - 3D - model.

The device is advantageously an apparatus, or a physical part of an apparatus, designed, configured and/or adapted for performing the mentioned functions and produce the mentioned effects or results. In alternative implementations, the device is embodied as a set of apparatus or physical parts of apparatus, whether grouped in a same machine or in different, possibly remote, machines. The device may for example have functions distributed over a cloud infrastructure and be available to users as a cloud-based service, or have remote functions accessible through an API ("application programming interface").

The device comprises a user graphical interface 2 including at least one display device 9 configured for displaying a virtual scene, that will be described more in details below, a controller 6 for interacting with the user graphical interface 2 and the virtual scene, and at least one processor 10 configured for processing data received from the controller 6 and for controlling the at least one display device 9, and for carrying out the method for assisting a user 4 positioning at least one graphical landmark within a three-dimensional - 3D - model.

### Controller 6

The controller 6 is configured so that a user 4 interacts with the user graphical interface 2. The user 4 can be a doctor or any other health professional.

The controller 6 may also be configured to acquire a current position of a predetermined user point A, of the user 4, in a predetermined user coordinate system 12.

The predetermined user coordinate system 12 is, for instance, fixed with respect to the user's environment. The predetermined user coordinate system 12 uses one or more numbers, or coordinates, to uniquely determine the position of points or other geometric elements in the user's environment.

The controller 6 may include a handheld motion tracking sensor, thereby allowing the user 4 to interact with the virtual scene using hand gestures. In this case, the user point A may be a point of the motion tracking sensor. The virtual scene may be represented in the user graphical interface 2, more specifically the virtual scene may be displayed on the at least one display device 9, which can be a 2D or 3D display device as will be described below. More in details, the controller 6 may be equipped with sensors (like accelerometers and gyroscopes) that enable the method to track its position and orientation in 3D space. The method may also relay on the use of external sensors or cameras to enhance tracking accuracy of the controller 6.

The controller 6 may also include other devices, such as a touch screen or a trackpad, a game controller, a mouse and/or a keyboard, thereby further allowing the user 4 to interact with the virtual scene as will be explained later.

The controller 6 may further include buttons and/or switches configured to allow the user 4 to interact with the virtual scene. Such buttons and/or switches may be included in the handheld motion tracking sensor, or may be included in a separate device of the controller 6, such as the touch screen, game controller, mouse and/or keyboard mentioned above.

Particularly, when the virtual scene is displayed on a 3D display device such as a virtual reality headset, the controller 6 may include a 3D controller or a joystick connected to the virtual reality headset.

Preferably, the controller 6 is configured to allow the user 4 to input (for example through the buttons and/or switches) specific instructions, such as an object display instruction, an object shifting instruction or a transformation instruction. Such instructions advantageously allow the user 4 to add and/or manipulate 3D objects in the virtual scene, as will be described below.

The controller 6 may also be configured to allow the user 4 to manipulate (e.g., to rotate and/or to zoom in on or out of) 3D objects in the virtual scene, and/or to change a direction along which the user 4 views the virtual scene displayed by the at least one display device 9.

Alternatively, or in addition, the controller 6 includes virtual buttons displayed in the virtual scene to allow the user 4 to interact with the virtual scene, and 3D objects in the virtual scene.

### Display device 9

As mentioned previously, the at least one display device 9 is configured to display the virtual scene, that will be described below.

The at least one display device 9 may include a screen, as shown on **Figure 1****.** The nature of the at least one display device 9 will depend on the embodiments.

In some embodiments, the at least one display device 9 may be a 2D display device such as: a computer monitor, a tablet, a smartphone, or a projection screen. Despite the inherent limitation of representing a three-dimensional object in a two-dimensional space, 2D display devices have many advantages, among, for example:
- Compatibility with existing hardware and software platforms. Displaying a 3D model on these devices allows for seamless integration into existing workflows without the need for specialized hardware or software.
- Accessibility to a broad audience, including healthcare professionals, patients, researchers, and educators. By presenting 3D models on familiar devices, such as desktop computers or mobile devices, information can be easily shared and accessed across different settings and user demographics.
- Cost-Effectiveness compared to specialized 3D display devices. This affordability enables broader adoption of 3D visualization technologies across healthcare organizations and educational institutions.
- Interactivity, even if 2D display devices lack the immersive capabilities of dedicated 3D displays, they still offer interactive features such as touchscreens, mouse input, and gesture controls. Users can interact with 3D models on 2D displays by rotating, zooming, and manipulating the view, enhancing their understanding and engagement with the content.

In some other embodiments, the at least one display device 9 may be a 3D display device such as a stereoscopic 3D monitor, an autostereoscopic 3D display, a virtual reality - VR - headset, an augmented reality - AR - device, or a 3D holographic display. One of the most significant advantages of 3D display devices is the ability to convey depth perception, providing users with a more realistic and immersive viewing experience, especially for the positioning of graphical landmark. Furthermore, by presenting volumetric data in three dimensions, users can visualize internal structures from multiple angles and perspectives, enabling a more comprehensive understanding of the patient's anatomy and pathology. Compared to 2D display devices, viewing a 3D model on a dedicated 3D display provides users with a better sense of spatial awareness and orientation. This enhanced spatial perception is particularly beneficial for surgical planning where precise anatomical localization and spatial relationships are critical.

### Processor 10

The at least one processor 10 is connected to each of the controller 6, the user graphical interface 2 and the at least one display device 9.

The at least one processor 10 corresponds, for example, to a workstation, a laptop, a tablet, a smartphone, programmable logical device (e.g., FPGA) for on-board calculation or a head-mounted display (HMD) such as a virtual reality headset.

As shown on **Figure 2**, the at least one processor 10 may comprise the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing due to their highly parallel structure;
- a non-volatile memory of ROM type 96;
- a RAM 97;
- a power supply 98; and
- a radiofrequency unit 99.

Alternatively, the power supply 98 is external to the at least one processor 10.

The controller 6 is, for instance, connected to at least part of the aforementioned modules, for instance through the bus 95.

Part of the user graphical interface 2 is, for instance, connected to at least part of the aforementioned modules, for instance through the bus 95.

The at least one display device 9 is connected to the graphics card 92, for instance through a suitable interface. For instance, a cable can be used for tethered transmissions, or the RF unit 99 can be used for wireless transmissions.

Each of memories 97 and 921 includes registers, which can designate in each of the memories, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner. Each of them does not necessarily correspond to adjacent memory locations and can be distributed otherwise (which covers notably the situation in which one register includes several smaller registers).

When switched-on, the microprocessor 91 loads and executes the instructions of the program 970 contained in the RAM 97 to allow operation of the visualization device 2 in the fashion described in the present disclosure.

As will be understood by a skilled person, the presence of the graphics card 92 is not mandatory, and can be replaced with entire CPU processing and/or other implementations.

### Operation

The device is configured to receive as input 18 a three-dimensional - 3D - model obtained from at least one 3D image. The 3D model is representative of at least one portion of a patient's body and comprises at least one anatomical element of interest on which the at least one graphical landmark is to be positioned.

The 3D model may also have been preliminary computed based on 2D images of the portion of the subject's body, such as CT scan ("Computed Tomography scan") images, PET ("Positron Emission Tomography") images, MRI ("Magnetic Resonance Imaging") images, or cone beam CT images. Alternatively, the device may be configured to receive as input 18 2D images of the portion of the subject's body, and then compute via the at least one processor 10, from the received 2D images, the 3D model.

The at least one processor is configured to compute a surface of the at least one anatomical element of interest, and to compute a first 3D rendering 20 of the surface by using a 3D rendering technique.

In some embodiments, computing the surface of the at least one anatomical element of interest comprises executing a segmentation algorithm for segmenting the surface of the at least one anatomical element of interest.

In some embodiments, the 3D rendering technique is a volume rendering technique, such as raycasting or raytracing.

As illustrated on **Figures 3A** (in color) and **3B** (in black and white), the user graphical interface 2 is configured:
- to display, on the at least one display device 9, a virtual scene comprising the first 3D rendering 20 of the surface on which is overlayed a virtual representation 30 of the controller 6; and
- to display on the surface a spot indicator 40 providing a visual representation of distances between one reference point on the surface, the reference point being selected by the user 4 using the controller 6, and surrounding pixels belonging to the surface.

Displaying the spot indicator 40 provides a local displaying enhancement of reliefs on the at least one anatomical element of interest represented by the surface.

In some embodiments, the spot indicator 40 on the surface is visualized by at least one displaying characteristic taken among: color, intensity, transparency, opacity. For example, in **Figures 3A** and **3B**, the surface corresponds to the surface of bones of a junction, on which three spot indicators 40 have been positioned. The shape and the intensity of those spot indicators 40 vary according to the depth and relied of the surface.

Particularly, in some embodiments where the virtual scene is display on a 2D display device, the at least one displaying characteristics of the spot indicator 40 may be calculated using a distance between the selected reference point on the surface and the surrounding pixels belonging to the surface within a predefined spot region approximately centered on the reference point.

For guiding the user 4 to identify anatomical landmarks of interest and positioning the at least one graphical landmark to a desired localization on the surface, the controller 6 is also configured to interactively move the spot indicator 40 on the surface. As illustrated in **Figures 3A** and **3B**, the user 4 can select, with the controller 6 a graphical landmark to move. To identify which graphical landmark is selected for moving, the spot indicator may vary and comprise, for example, a sort of shadow or transparent spot indicator superimposed, as it is visible at the end of the visual representation of the controller 6 on **Figures 3A** and **3B****.**

In some embodiments, the reference point is further positioned on the surface at an intersection between a line, passing between a virtual position associated to the user's eyes in the virtual scene and the virtual representation 30 of the controller 6, and the surface.

In some embodiments where the virtual scene is display on a 2D display device and the surface has been segmented, the surface may be represented by a mesh composed of vertices, edges, and faces defining the at least one portion of the at least one anatomical element of interest shape. In this case, selecting the reference point on the surface using the controller 6 may comprise:
- calculating a position of the virtual representation 30 of the controller 6 in the virtual scene;
- identifying a vertex of the mesh as being the closest vertex to the calculated position of the controller's virtual representation 30;
- selecting the identified vertex as the reference point on the surface

In some embodiments where the virtual scene is display on a 3D display device, computing the surface may comprise executing a ray casting or ray tracing algorithm on the at least one 3D image to obtain a 3D patient rendering, and a depth map and/or a position map associated to the 3D patient rendering, the surface being obtained based on the depth map and/or the position map, and wherein displaying on the surface the spot indicator 40 depends on the depth map and/or the position map.

Still in some embodiments where the virtual scene is display on a 3D display device, the at least one displaying characteristic of the spot indicator 40 may be obtained by including a local light source during computation of 3D rendering, by:
- determining a current position of the local light source from the reference point selected by means of the controller 6;
- computing a current 3D rendering of the surface considering the local light source.

In this case, as the reference point selected by means of the controller 6 changes as a result of the interactions of the user 4 with the controller 6, the current 3D rendering is recomputed taking into account the current position of the local light source.

Advantageously, when the virtual scene is displayed on a 3D display the virtual representation 30 of the controller 6 may be an 3D object, such as a 3D cross or a 3D landmark, which is more adapted to 3D view.

In some embodiments, the device further comprises a memory (e.g. memories 97 and 921 of **Figure 2**) configured to record the coordinates corresponding to the position of the at least one graphical landmark on the 3D model into a memory. Advantageously, these recorded coordinates can be used for positioning a medical device, such as a surgical device, an imaging device or an implant as relative to these coordinates during a medical intervention.

In some embodiments, the at least one processor is further configured to compute measurements using the coordinates of at least two graphical landmarks.

In some embodiments, the at least one processor 10 is further configured to receiving initial predefined localization of at least one of the anatomical landmarks of interest associated to the 3D model, and the user graphical interface 2 is further configured to display, in the virtual scene, on the surface at least one guiding landmark corresponding to the initial predefined localization. In this case, the controller 6 is configured to move the at least one guiding landmark onto another localization on the surface.

**Figure 4** is an example of flow chart of the method for assisting a user positioning at least one graphical landmark within a three-dimensional - 3D - model by means of a user graphical interface and a controller configured so that the user interacts with the user graphical interface. As previously described, the 3D model is obtained from at least one 3D image, and is representative of at least one portion of a patient's body comprising at least one anatomical element of interest on which the at least one graphical landmark is to be positioned.

In a step S30, a surface of the at least one anatomical element of interest is computed.

In a step S40, a first 3D rendering of the surface by using a 3D rendering technique is computed.

In a step S50, a virtual scene is displayed in the user graphical interface. The virtual scene comprises the first 3D rendering of the surface on which is overlayed a virtual representation of the controller.

In a step S60, a spot indicator is displayed on the surface, in the virtual scene. The spot indicator provides a visual representation of distances between one reference point on the surface and surrounding pixels belonging to the surface. The reference point is selected by the user using the controller. Displaying the spot indicator provides a local displaying enhancement of reliefs on the at least one anatomical element of interest represented by the surface.

As described before, the controller is configured to interactively move the spot indicator on the surface, as guidance for the user to identify anatomical landmarks of interest, and to position the at least one graphical landmark to a desired localization on the surface. Thus, the method may further comprise a step S70 where the spot indicator is moved until a desired localization.

In some embodiments and as previously described, the method may also comprise optional steps, such as:
- a receiving step S10 wherein at least one 3D image (or respectively 2D images) of the portion of the subject's body is received; and
- a computing step S20 wherein the 3D model representative of the at least one portion of a patient's body and comprising the at least one anatomical element of interest is computed based on the at least one 3D image (respectively the 2D images) received, before computing the surface of the at least one anatomical element of interest.

Advantageously, the method may further comprise a step S80 wherein the coordinates corresponding to the position of the at least one graphical landmark on the 3D model are recorded into a memory. This will enable the user 4 to export the coordinates of the graphical landmarks positioned, during the planning of a medical operation for example, within an exported file, so they can be retrieved by the user 4 during a second planning session.

In some embodiments, the method may also comprise a step S90 wherein recorded coordinates corresponding to at least one position of at least one registered graphical landmark (which may have been recorded during a previous planning session by the user or another health professional) on the 3D model are loaded, so that a corresponding spot indicator may be displayed on the first 3D rendering in the virtual scene by the user graphical interface 2.

The preferential and advantageous characteristics linked to the device previously described are also applicable to the method.

### EXAMPLES

The present invention is further illustrated by the following examples.

### Example 1: point light source moved by a mouse on a mesh

In this embodiment, as illustrated in **Figures 5A** (in color) and **5B** (in black and white), a spot indicator represented by a point light source is present on a surface mesh object that can be clicked on with a mouse and dragged along the surface, as illustrated in **Figures 6A** (in color) and **6B** (in black and white):
- interaction is done by means of ray cast perpendicular to the mouse cursor position to the point light source which has a spherical collider associated to it;
- clicking and holding the mouse when it is above the spherical collider enables it to be dragged along the surface;
- when dragging the mouse, subsequent ray casts are performed towards the collider of the mesh to detect which is the closest triangle that the mouse cursor is above;
- the point light source is displaced to the position of this closest triangle on the mesh, hence remaining on the manifold of the surface mesh;
- as the point light source moves as does its lighting effects on the mesh.

### Example 2: point light source moved by a gizmo on a mesh

In this embodiment, as illustrated in **Figures 7A** (in color) and **7B** (in black and white), a first spot indicator represented by a point light source (cf. the blue sphere) is present on a surface mesh object whose position along the surface can be changed with a discrete manipulation gizmo (cf. the four arrows, two in blue to move the first spot indicator which is selected along a first line, and two in red to move the first spot indicator along a second line orthogonal to the first one):
- a manipulation gizmo is associated with the point light source on the surface of the mesh;
- clicking on any of the direction arrows associated to gizmo will move the point light source at a predefined distance increment in the prescribed direction;
- the point light source is placed on the vertex closest to this incremented direction, hence remaining on the surface of the mesh;
- as the light source moves as does its lighting effects on the mesh.

Furthermore, these **Figures 7A** and **7B** show that anatomical measurements can be made between at least two graphical landmarks, like between the first spot indicator and a second spot indicator (cf. the red dot above the blue sphere) indicated as being 16 mm away. The result of the anatomical measurement can also be displayed on the first 3D rendering displayed in the virtual scene.

### Example 3: color/heat-map + proximity sensor

In this embodiment, as illustrated in **Figures 8A** (in color) and **8B** (in black and white), a spot indicator represented by a point light source can be moved with the controller on the 3D first rendering displayed in the virtual scene, and depending on the position of the spot indicator on the surface and especially on the surface's depth and relief, the local coloring of the surface is changed based on the proximity of the landmark (represented by the spot indicator). To do so:
- in a shader program, the distance between each rasterized pixel of the mesh object and the landmark is calculated;
- the color of the rasterized pixel is modulated based on this calculated distance
- if the landmark moves, as does the local modulation of the color on the surface of the mesh.

## Claims

1. A computer-implemented method for assisting a user (4) positioning at least one graphical landmark within a three-dimensional - 3D - model by means of a user graphical interface (2) and a controller (6) configured so that the user interacts with said user graphical interface (2), said 3D model being obtained from at least one 3D image and being representative of at least one portion of a patient's body and comprising at least one anatomical element of interest on which the at least one graphical landmark is to be positioned, said method comprising:
- computing a surface of said at least one anatomical element of interest (S30);
- computing a first 3D rendering (20) of said surface by using a 3D rendering technique (S40);
- displaying, in the user graphical interface (2), a virtual scene comprising said first 3D rendering (20) of said surface on which is overlaid a virtual representation (30) of said controller (S50);
- in said virtual scene, displaying on said surface a spot indicator (40) providing a visual representation of distances between one reference point on said surface, said reference point being selected by the user (4) using said controller (6), and surrounding pixels belonging to the surface, wherein displaying said spot indicator (40) provides a local displaying enhancement of reliefs on the at least one anatomical element of interest represented by the surface (S60);
wherein said controller (6) is further configured to interactively move said spot indicator (40) on said surface, as guidance for the user (4) to identify anatomical landmarks of interest, and to position said at least one graphical landmark to a desired localization on said surface.

2. The method according to claim **1**, wherein the 3D rendering technique is a volume rendering technique, such as raycasting or raytracing.

3. The method according to claim **1** or **2**, wherein the reference point is further positioned on said surface at an intersection between a line, passing between a virtual position associated to the user's eyes in the virtual scene and the virtual representation (30) of said controller (6), and said surface.

4. The method according to any one of claims **1** to **3**, wherein computing the surface of said at least one anatomical element of interest (S30) comprises executing a segmentation algorithm for segmenting the surface of said at least one anatomical element of interest.

5. The method according to any one of claims **1** to **4**, wherein the spot indicator (40) on the surface is visualized by at least one displaying characteristic taken among: color, intensity, transparency, opacity.

6. The method according to any one of claims **1** to **5**, wherein the virtual scene is displayed on 2D display device such as: a computer monitor, a tablet, a smartphone, or a projection screen.

7. The method according to claim **6** depending on claim **5**, wherein the at least one displaying characteristics of the spot indicator (40) is calculated using a distance between the selected reference point on said surface and the surrounding pixels belonging to the surface within a predefined spot region approximately centered on the reference point.

8. The method according to claim **6** or **7** and depending on claim **4**, said surface is represented by a mesh composed of vertices, edges, and faces defining a shape of said at least one anatomical element of interest, and selecting said reference point on said surface using said controller (6) comprises:
- calculating a position of the virtual representation (30) of said controller (6) in the virtual scene;
- identifying a vertex of said mesh as being the closest vertex to said calculated position of the controller's virtual representation (30);
- selecting said identified vertex as the reference point on said surface.

9. The method according to any one of claims **1** to **5**, wherein the virtual scene is displayed on 3D display device such as: a stereoscopic 3D monitor, an autostereoscopic 3D display, a virtual reality - VR - headset, an augmented reality - AR - device, or a 3D holographic display.

10. The method according to claim **9**, wherein computing said surface (S30) comprises executing a ray casting or ray tracing algorithm on said at least one 3D image to obtain a 3D patient rendering, and a depth map and/or a position map associated to said 3D patient rendering, said surface being obtained based on the depth map and/or the position map, and wherein displaying on said surface said spot indicator (40) depends on said depth map and/or said position map.

11. The method according to claim **9** or **10** and depending on claim **5**, wherein the at least one displaying characteristic of the spot indicator (40) is obtained by including a local light source during computation of 3D rendering, by:
- determining a current position of said local light source from the reference point selected by means of said controller (6);
- computing a current 3D rendering of said surface considering said local light source;
wherein, as the reference point selected by means of said controller (6) changes as a result of the interactions of the user (4) with the controller (6), the current 3D rendering is recomputed taking into account the current position of the local light source.

12. The method according to any one of claim **9** to **11**, wherein the virtual representation (30) of the controller (6) is a 3D object, such as a 3D cross or a 3D landmark.

13. The method according to any one of claim **1** to **12**, further comprising recording the coordinates corresponding to the position of said at least one graphical landmark on the 3D model into a memory (S80).

14. The method according to any one of claim **1** to **13**, further comprising computing measurements using the coordinates of at least two graphical landmarks.

15. The method according any one of claims **1** to **14**, further comprising:
- receiving initial predefined localization of at least one of said anatomical landmarks of interest associated to said 3D model;
- displaying, in the virtual scene, on said surface at least one guiding landmark corresponding to said initial predefined localization;
wherein the controller (6) is configured to move said at least one guiding landmark onto another localization on the surface.

16. Use of recorded coordinates with the method according to claim **13**, for positioning a medical device, such as a surgical device, an imaging device or an implant as relative to these coordinates during a medical intervention.
